# EUROPÄISCHE PATENTSCHRIFT

(11) **EP 3 401 411 B1**
(45) Veröffentlichungstag und Bekanntmachung des Hinweises auf die Patenterteilung: **20.07.2022**
(21) Anmeldenummer: 18171147.4
(22) Anmeldetag: 08.05.2018
(51) Int. Cl.: G01N 21/89, G01N 21/88, G06T 7/586, D06H 3/08, C14B 17/00, G01N 33/44, G06T 7/00

(54) **VERFAHREN UND VORRICHTUNG ZUR FEHLERSTELLENERKENNUNG BEI BIEGESCHLAFFEN KÖRPERN, INSBESONDERE TIERHÄUTEN**
METHOD AND APPARATUS FOR DETECTING DEFECT SITES IN FLEXIBLE BODIES, IN PARTICULAR ANIMAL SKINS
PROCÉDÉ ET DISPOSITIF DE DÉTECTION DES DÉFAUTS DANS DES CORPS SOUPLES, EN PARTICULIER PEAUX D'ANIMAUX

(30) Priorität: 11.05.2017 DE 102017004519
(43) Veröffentlichungstag der Anmeldung: 14.11.2018
(73) Patentinhaber: Capex Invest GmbH, 33607 Bielefeld (DE)
(72) Erfinder: Bruder, Manuel, 33605 Bielefeld (DE)
(74) Vertreter: Roche, von Westernhagen & Ehresmann

(56) Entgegenhaltungen:
- DE-A1- 10 207 574
- DE-A1-102013 005 489
- US-A1- 2015 355 101

## Beschreibung

Die Erfindung betrifft zunächst ein Verfahren zur (automatischen) Fehlerstellenerkennung bei biegeschlaffen Körpern, nämlich insbesondere bei Tier- oder Lederhäuten.

So werden aus Lederhäuten maschinelle Bearbeitungsstücke herausgeschnitten, welche dann typischerweise im Möbelpolster- oder Kfz-Sitzbereich Verwendung finden.

Die Tierhäute weisen vor dem Schneidevorgang naturgemäß Fehlerstellen oder Fehlerbereiche auf, welche beispielsweise durch Narben, Insektenstiche, Löcher, Falten oder Ähnliches hervorgerufen werden können. Diese Fehlerstellen müssen erkannt bzw. markiert werden, so dass während des anschließenden Schneidprozesses um diese ungewünschten Fehlerstellen herumgeschnitten werden kann. Hierdurch liegen die Fehlerstellen dann nicht im Bereich der weiterzuverarbeitenden Verarbeitungsstücke.

Aus dem Stand der Technik ist es zunächst hinlänglich bekannt, derartige Fehlerstellen manuell zu erkennen und dann mit Hilfsmitteln zu markieren, so z.B. aus der DE 35 21 357 A1 oder der DE 42 31 222 C1. Auch ist es bekannt, Fehlerstellen in digitalen Aufnahmen der Haut an einem Display mit Hilfe eines Eingabegerätes manuell zu kennzeichnen. Ein sogenanntes Nesting-Programm kann die Schnittmuster unter Berücksichtigung der eingegebenen oder markierten Fehlerstellen dann so auf der Haut platzieren, dass die Fehlerstellen nicht in den zu schneidenden Mustern liegen.

Auch wenn derartige manuelle Erkennungs- oder Markierungsverfahren grundsätzlich zuverlässig sind, besteht ein stetes Bestreben der Fachwelt, die Fehlererkennung automatisch bzw. automatisiert durchzuführen.

Ein derartiges automatisiertes Verfahren zur Fehlerstellenerkennung ist beispielsweise aus der DE 10 2013 005 489 A1 der Anmelderin bekannt, wobei derartige Verfahren zunächst eine Digitalisierung der Tierhaut oder zumindestens von entsprechenden Bereichen umfassen.

Die automatische Fehlerstellenerkennung erfolgt dann beim genannten Stand der Technik auf Basis der Auswertung der digitalen Aufnahmen bzw. deren Helligkeits- oder Graustufen- bzw. Farbwerten. Auf diese Werte werden Algorithmen angewendet, teilweise unter Berücksichtigung von Nachbarschaftsbeziehungen der einzelnen Bildpunkte untereinander.

Derartige Verfahren stellen bereits sehr zufriedenstellende Ergebnisse bereit, wobei eine weitere Optimierung der Ergebnisse naturgemäß stets gewünscht ist und insbesondere auch eine Verbesserung der Nutzerfreundlichkeit der (automatisierten) Verfahren.

Die Aufgabe der vorliegenden Erfindung besteht demnach darin, ein Verfahren bereitzustellen, welches eine sehr zuverlässige, insbesondere automatische, Fehlerstellenerkennung ermöglicht, die weiter auch besonders nutzerfreundlich ist.

Die vorliegende Erfindung löst die gestellte Aufgabe mit den Merkmalen des Patentanspruches 1.

Die Erfindung besteht demnach im Wesentlichen darin, anstelle der aus dem Stand der Technik bekannten Auswertung von Helligkeitswerten pro Flächeneinheit vielmehr die Orientierung oder Ausrichtung dieser Flächeneinheiten bei der Fehlerstellenerkennung zu berücksichtigen bzw. auszuwerten. Mit anderen Worten werden anstatt der mehr oder weniger eindimensionalen Helligkeitswerte nunmehr 3D-Angaben bei der automatischen Fehlerstellenerkennung verarbeitet.

Diese 3D-Daten oder -werte können hierbei beispielsweise durch die Flächennormale eines entsprechenden Flächenkleinstbereiches angegeben werden.

Hierbei kann als Kleinstbereich im Sinne der vorliegenden Erfindung beispielsweise der Bereich aufgefasst werden, welcher durch einen Pixel der Aufnahmeeinrichtung abgebildet wird. Vorzugsweise kann in diesem Sinne für jeden Pixel einer Aufnahme eine Ausrichtungsinformation, insbesondere eine Flächennormale, ermittelt und vorzugsweise weiterverarbeitet werden. Lediglich der Vollständigkeit halber sei angemerkt, dass selbstverständlich auch - insbesondere abhängig von der Auflösung der Aufnahmeeinrichtung - Ausrichtungsinformationen (bzw. Normalenvektoren) berechnet und weiterverarbeitet werden können, welche einem Kleinstbereich zugeordnet sind, der in den Aufnahmen aus mehreren Pixeln besteht. Zu große Bereiche oder Flächen sollten allerdings naturgemäß nicht verwendet werden, da die Oberflächenausrichtung des Körpers natürlich für eine möglichst genaue Fehlerstellenerkennung hinreichend abgebildet werden sollte.

Grundsätzlich können die berechneten Ausrichtungsinformationen für die Kleinstbereiche dann mit (an den 3D-Modus angepassten) Algorithmen ausgewertet werden, welche ähnlich aus dem Stand der Technik für die Helligkeitswerte bereits bekannt sind (Wavelet-Filterung oder Ähnliches).

Die Berechnung der Ausrichtungsinformationen beruht dabei auf der Auswertung von mehreren Aufnahmen, welche bei unterschiedlichen Beleuchtungssituationen getätigt werden. Da die Position der unterschiedlichen Leuchten oder Lichtquellen ebenso bekannt ist wie die Position der Aufnahmeeinrichtung, können durch die bei unterschiedlichen Beleuchtungssituationen entstandenen Aufnahmen Rückschlüsse auf die Ausrichtung (bzw. die durchschnittliche Ausrichtung) eines Kleinstbereiches des Körpers gezogen werden (wie dies beispielsweise geschehen kann, wird später noch genauer erläutert).

Jedenfalls werden diese Ausrichtungsinformationen (anstelle der Helligkeits- oder Graustufenwerte des Standes der Technik) im weiteren Verfahren dazu verwendet, die Fehlerstellen des Körpers zu ermitteln. Diese Analyse kann insbesondere automatisch erfolgen, vorzugsweise mit Hilfe einer Recheneinheit (einer entsprechenden Vorrichtung zur automatischen Fehlerstellenerkennung).

Ein entscheidender Vorteil des erfindungsgemäßen Verfahrens liegt hierbei darin, dass die Analyse zur Erkennung von Fehlerstellen des Körpers im Wesentlichen farbunabhängig erfolgen kann. Da Ausrichtungsinformationen verwertet werden, spielen Farbunterschiede oder Farben des biegeschlaffen Körpers nicht eine so große Rolle wie bei den Verfahren des Standes der Technik, welche mit Farbwerten oder Ähnlichkeitswerten arbeiten. Dies erscheint unmittelbar einleuchtend. Mit anderen Worten: Farbunterschiede des Körpers, welche auf das Vorhandensein von Fehlerstellen in der Regel keinen Einfluss haben, können bei der Analyse im Wesentlichen ignoriert werden, was zu besseren Ergebnissen der Fehlerstellenerkennung führt.

Die mehreren, oben genannten Aufnahmen, welche zur Berechnung der Ausrichtungsinformationen genutzt werden, werden dabei bei unterschiedlichen Beleuchtungssituationen getätigt. Hierzu sind in der Regel mehrere Lichtquellen vorgesehen, welche durch eine, insbesondere vorprogrammierte, Schaltung derart aktiviert werden, dass pro durchgeführter Aufnahme jeweils eine andere Konstellation an Lichtquellen aktiv ist. Im einfachsten Fall kann pro Aufnahme genau eine der Lichtquellen aktiv und die anderen abgeschaltet sein. Grundsätzlich ist es aber auch vorstellbar, die Aktivierung der Lichtquellen zu mischen.

Entscheidend ist, dass in dem Verfahren die Aktivierung der Lichtquellen auf die Durchführung der Aufnahmen (durch die Aufnahmeeinrichtung) abgestimmt ist. Pro Aufnahme des Körpers oder Körperbereiches liegt also eine andere Schaltung der Lichtquellen vor.

Die mehreren Aufnahmen werden demnach zeitlich nicht gleichzeitig, sondern versetzt oder nacheinander getätigt.

Weiter können die mehreren Aufnahmen typischerweise einen Teilbereich des Körpers betreffen oder in einem anderen Ausführungsbeispiel auch den gesamten Körper. In diesem Sinne macht die Aufnahmeeinrichtung also zeitlich versetzt mehrere Aufnahmen eines Teilbereichs des Körpers oder des gesamten Körpers.

Hierbei kann die Aufnahmeeinrichtung selbstverständlich auch mehrere Einheiten, wie beispielsweise mehrere CCD- oder Digitalkameras, umfassen. Eine derartige Ausgestaltung ermöglicht ein komfortableres Verfahren zur Fehlerstellenerkennung, da gleichzeitig ein größerer Bereich des zu untersuchenden Körpers aufgenommen werden kann. So können für eine erfindungsgemäße einzelne Aufnahme insbesondere die gleichzeitig aufgenommenen Bilder mehrerer Kameras zusammengesetzt werden. Zeitlich versetzt können dieselben Kameras dann gleichzeitig jeweils ein weiteres Bild aufnehmen, welches dann zu einer zweiten Aufnahme zusammengesetzt wird. Die erste und zweite Aufnahme und anschließend noch mehrere Aufnahmen ermöglichen dann Rückschlüsse über die Ausrichtungsinformationen der Kleinstbereiche des Körpers.

Mehrere Kameras können hierbei insbesondere in einer Reihe angeordnet sein (beispielsweise mindestens zwei Kameras, vorzugsweise drei oder mehr Kameras). Die Kameras können dabei so ausgerichtet sein, dass die abzubildenden Bereiche (bzw. ihre Abbildungen) sich nicht oder kaum überschneiden. Selbstverständlich kann theoretisch auch eine gitterartige Anordnung von mehreren Kameras vorgesehen sein.

Das gleiche Ziel der Verwendung mehrerer Kameras kann aber beispielsweise auch dadurch erreicht werden, dass eine einzelne (Digital-) Kamera vorgesehen ist, welche zwischen verschiedenen Positionen verstellbar oder verfahrbar ist. Auf diese Weise kann eine Aufnahme eines (großen) Bereiches oder Abschnittes eines Köpers oder des ganzen Köpers auch beispielsweise dadurch erreicht werden, dass eine Kamera bei jeweils gleicher Beleuchtungssituation Teilaufnahmen in unterschiedlichen Positionen tätigt, typischerweise also zeitversetzt nacheinander an unterschiedliche Orte verfährt oder verstellt wird. Die auf diese Weise nacheinander aufgenommenen Teilaufnahmen können auch zu einer gewünschten Aufnahme zusammengesetzt werden, die dann einen größeren Bereich abbildet als eine einzelne Teilaufnahme.

Wie oben beschrieben kann in einer bevorzugten Ausführungsform ein Teilbereich des Körpers aufgenommen werden oder alternativ der ganze Körper. In beiden Fällen sollte der Körper vorzugsweise beweglich relativ zur Aufnahmeeinrichtung und/oder den Lichtquellen gelagert sein. Dies kann beispielsweise mit Hilfe eines Förderbandes erfolgen, welches den Körper (insbesondere abschnittsweise) in den Bereich der Aufnahmeeinrichtung und/oder der Lichtquellen hineintransportieren (und gegebenenfalls auch wieder heraustransportieren) kann. Alternativ könnten selbstverständlich auch die Aufnahmeeinrichtung und/oder die Lichtquellen (insbesondere gemeinsam) verlagerbar sein, vorzugsweise relativ zu einer festen Auflagefläche für den Körper.

In beiden genannten Fällen ist es dabei von Vorteil, dass die Aufnahmeeinrichtung und/oder die Lichtquellen von einer haubenartigen Einrichtung abgedeckt sind. Dies sorgt dafür, dass die Aufnahmen bei einer klar definierten Lichtsituation erfolgen können. Die Position der Lichtquellen ist bekannt. Würde Umgebungs- oder Außenlicht auf den aufzunehmenden Teilbereich des Körpers gelangen, wäre eine Ermittlung der Ausrichtungsinformationen naturgemäß kaum noch möglich.

In diesem Sinne ist der aufzunehmende (Teil-) Bereich des Körpers für eine Aufnahme abgedunkelt (mit Ausnahme der geschalteten, vordefinierten, positionsbekannten Lichtquelle).

Bei der Lichtquelle sollte es sich im Sinne der Erfindung vorzugsweise um eine Punktlichtquelle handeln, also beispielsweise nicht um eine Stablichtquelle (wie eine Leuchtstoffröhre) oder Ähnliches. Die Punktlichtquelle bietet hierbei den Vorteil einer besseren Berechnung von Ausrichtungsinformationen der Kleinstbereiche des Körpers, da das entsprechende Berechnungsverfahren erleichtert wird. Geht das Licht lediglich von im Wesentlichen einem Punkt aus, kann das System genaue(re) Rückschlüsse auf die Ausrichtung des reflektierenden Kleinstbereiches tätigen. Vorzugsweise sind die Lichtquellen (oder jedenfalls ein Teil der Lichtquellen) symmetrisch um die Aufnahmeeinrichtung bzw. um einen Teil der Aufnahmeeinrichtung (wie beispielsweise eine Kamera) herum angeordnet. In diesem Sinne können beispielsweise drei Kameras der Aufnahmeeinrichtung in einer Reihe angeordnet sein, wobei jede der Kameras zu vier Lichtquellen benachbart ist (diese vier Lichtquellen können die Kamera ringartig umgeben). Sind die Lichtquellen entsprechend angeordnet, kann somit für drei Kameras eine Lichtquellenanordnung von beispielsweise zehn Lichtquellen genutzt werden.

Erfindungsgemäß handelt es sich typischerweise um mehrere Lichtquellen, wobei jede Lichtquelle eine andere Beleuchtungssituation erzeugen kann. Diese Annahme geht zunächst davon aus, dass Lichtquellen grundsätzlich nicht in ihrer Position verstellbar sind. Von der Erfindung umfasst sind selbstverständlich aber auch Systeme, bei welchen die Lichtquellenposition verstellt werden kann. In diesem Sinne ist es sogar vorstellbar, dass für das erfindungsgemäße Verfahren lediglich eine einzige Lichtquelle genutzt wird, welche zur Durchführung der besagten Aufnahmen in unterschiedliche Positionen überführt wird. Auf diese Weise können die Aufnahmen bei unterschiedlichen Beleuchtungssituationen entstehen, obwohl tatsächlich nur eine Lichtquelle verwendet wird. Entscheidend ist hierbei, dass die Beleuchtung für die unterschiedlichen Aufnahmen aus unterschiedlichen Winkeln bzw. aus unterschiedlichen Lichtquellenpositionen erfolgt.

Die Lichtquelle(n) und die Aufnahmeeinrichtung sind hierbei vorzugsweise innerhalb besagter Haube angeordnet.

Die Lichtquellen bescheinen bei ihrer Aktivierung den im Haubenbereich befindlichen Teilbereich des Körpers.

Bei einem biegeschlaffen Körper handelt es sich im Sinne der Erfindung vorzugsweise um eine Tierhaut, um ein Leder oder Ähnliches.

Aus gattungsfremden Stand der Technik gemäß der US 2015/355101 A1 ist die Berechnung von Normalvektoren bezüglich der Oberfläche von Werkstücken bereits bekannt, nämlich auf dem Gebiet der Erfassung von Münzen und Etiketten. Dort werden Normalenvektoren allerdings hauptsächlich zur Bildbearbeitung für eine bessere Lesbarkeit verwendet. Auch wenn dort ein Erkennen von Fehlern offenbart ist, so ist dies doch nicht näher beschrieben.

Nachdem mit dem erfindungsgemäßen Verfahren die Fehlerstellen eines biegeschlaffen Körpers hinreichend erkannt wurden (und gegebenenfalls sogar klassifiziert und/oder qualifiziert werden konnten), können die zu den Fehlerstellen ermittelten Daten (wie beispielsweise Lage oder Größe oder Art der Fehlerstelle) in einer Datenbank oder einer Recheneinheit zur späteren Verwendung gespeichert werden. Bei dieser weiteren Verwendung kann es sich beispielsweise um einen anschließenden, herkömmlichen Nesting-Prozess handeln, wobei eine Recheneinheit der Vorrichtung zur automatischen Fehlerstellenerkennung die auszuschneidenden Teile derart auf dem biegeschlaffen Körper platzieren kann, dass die Fehlerstellen dabei berücksichtigt werden.

Anschließend kann der biegeschlaffe Körper dann gemäß den Ergebnissen dieses Nesting-Prozesses bearbeitet, insbesondere zurechtgeschnitten werden. Diese Bearbeitung kann vorzugsweise automatisch erfolgen, insbesondere auch mit der Vorrichtung zur automatischen Fehlerstellenerkennung. Hierbei kann die Vorrichtung zur automatischen Fehlerstellenerkennung neben der Aufnahmeeinrichtung beispielsweise auch eine Schneideinheit (und/oder sogar einen - separaten - Schneidetisch) enthalten, welche mit der Steuerung oder Recheneinheit der Vorrichtung verbunden ist und von dieser steuerbar ist. Auch eine Projektionseinheit zur Projektion der ermittelten Fehlerstellen und/oder dem berechneten Nesting-Ergebnis auf den Körper kann umfasst sein.

Mit dem Verfahren zur automatischen Fehlerstellenerkennung können neben typischen Fehlerstellen, wie beispielsweise Narben, Mastfalten, Flämen, Insektenstichen, Schmutzeinschlüssen, Kratzern, Warzen, Gerbfehlern, Aushebern, Raustellen, Adern, Brandmarken, Falten (insbesondere Mastfalten) oder sonstigen unerwünschten Strukturen aber beispielsweise auch die Ränder des biegeschlaffen Körpers oder Löcher im biegeschlaffen Körper erkannt werden.

Durch die automatische Fehlerstellenerkennung wird es dabei überflüssig, den biegeschlaffen Körper vor der automatischen Analyse manuell bzw. händisch zu markieren. Fluoreszierende Farbe, eine digitale manuelle Markierung oder Ähnliches sind daher verzichtbar.

Vorteilhafterweise erfolgt die Berechnung der Ausrichtungsinformationen, also beispielsweise der Normalvektoren, für einen Kleinstbereich nach Art eines fotometrischen Stereoverfahrens: Dieses ist aus der Bildtechnik grundsätzlich bekannt und beschreibt ein Verfahren zur Abschätzung der Flächennormalen von Objekten, wobei das Objekt unter unterschiedlichen Lichtverhältnissen beobachtet bzw. aufgenommen wird. Das Verfahren basiert auf der Annahme, dass die Intensität oder Helligkeit des reflektierten Lichtes einer Fläche abhängig ist von der Ausrichtung oder Orientierung der Fläche im Verhältnis zur Lichtquelle und der Aufnahmeeinrichtung. Indem man die Lichtintensität misst, welche in eine Aufnahmeeinrichtung oder Kamera reflektiert wird, wird die Anzahl der Ausrichtungsmöglichkeiten der entsprechenden Oberfläche limitiert. Werden genug Aufnahmen bei unterschiedlichen Lichtsituationen (jeweils bei Aktivierung einer Lichtquelle aus einem unterschiedlichen Winkel) bereitgestellt, kann die Anzahl der möglichen Oberflächenausrichtungen auf eine einzelne Ausrichtung begrenzt werden, so dass die tatsächliche, zumindest durchschnittliche Ausrichtung der Fläche berechnet werden kann.

Mit Hilfe dieses Verfahrens können insbesondere die Flächennormalen für jeden Kleinstbereich berechnet werden. Von der Erfindung kann insbesondere auch umfasst sein, dass eine entsprechende Normalenkarte ("normal map") angezeigt wird. Eine solche "normal map" stellt dann für den Bereich der Aufnahmen eine typischerweise farbliche Darstellung bereit, welche anhand unterschiedlicher Farbwerte Rückschlüsse auf die an jedem Pixel der Darstellung vorgesehenen Normalenvektoren erlaubt. Die Anzeige einer solchen "normal map" ist lediglich optional zu verstehen. Typischerweise wird eine derartige "normal map" überhaupt nicht ausgegeben. Vielmehr wird tatsächlich für jeden Pixel bzw. jeden Kleinstbereich ein Normalenvektor berechnet und die entsprechende Karte (in welcher jedem Pixel eine Normale zugeordnet ist) wird dann vielmehr zur Analyse der Ausrichtungsinformation weiterverarbeitet.

Gemäß einer vorzugsweisen Ausgestaltung der Erfindung erfolgt die Analyse der Ausrichtungs- oder Orientierungsinformationen zur Erkennung der Fehlerstellen des Körpers durch ein intelligentes System. Als solch ein intelligentes System wird insbesondere ein neuronales Netzwerk verstanden (beispielsweise ein "convolution neural network").

Derartige neuronale Netzwerke werden auch als künstliches neuronales Netzwerk bezeichnet.

Es handelt sich hierbei um ein Netz aus künstlichen Neuronen, welches ein biologisches Vorbild hat. Es kann eine für den Anwendungsfall geeignete Topologie des Netzes gewählt oder vorbereitet werden, wobei ein derartiges Netz insbesondere "lernfähig" ist. Unter einem Lernprozess versteht man in diesem Zusammenhang das (automatische) Ändern von Gewichtungen, beispielsweise zwischen das Netz ausbildenden Algorithmen. Unter Umständen können auch neue Verbindungen erstellt oder bestehende Verbindungen von dem intelligenten System gelöscht werden. Künstliche neuronale Netze zeichnen sich insbesondere durch iterative oder rekursive Vorgehensweisen aus.

Vorzugsweise wird das intelligente System, insbesondere das neuronale Netz, erfindungsgemäß trainiert. Hierzu können dem intelligenten System Testdatensätze zugeführt werden, wobei die Testdatensätze von dem System auf Fehlerstellen untersucht werden. Damit das System beurteilen kann, wie das hierbei entstandene Ergebnis zu bewerten ist, wird dem System ebenfalls - für jeden Testdatensatz - ein zugehöriger Ergebnisdatensatz zugeführt. Testdatensatz und zugehöriger Ergebnisdatensatz können dann vom System abgeglichen oder verglichen werden, und das System kann auf diese Weise lernen, wie gut seine einzelnen Algorithmen geeignet sind, bestimmte Fehlerstellen zu erkennen.

Ergibt sich hierbei, dass bestimmte Algorithmen besser geeignet sind, können diese höher gewichtet werden. Hierzu kann das intelligente System das Ergebnis für einen Testdatensatz, beispielsweise Pixel für Pixel, mit dem zugehörigen Ergebnisdatensatz vergleichen. Sind die Abweichungen zwischen Ergebnis und Ergebnisdatensatz sehr groß, kann das System denselben Prozess beispielsweise mit einer anderen Gewichtung durchlaufen und überprüfen, ob mit der anderen Gewichtung bessere Ergebnisse herauskommen.

Anstelle einer anderen Gewichtung oder zusätzlich können beispielsweise auch andere Verbindungen im neuronalen Netzwerk geknüpft werden oder testweise gelöscht werden. Das System kann auf diese Weise lernen, welche Gewichtungen und/oder Verknüpfungen geeignet sind, zuverlässige Ergebnisse zur Fehlerstellenerkennung zu liefern. Das System kalibriert sich in diesem Sinne selbst.

Die entsprechenden Ergebnisdatensätze können dabei beispielsweise auf manueller Ergebniserkennung beruhen. In jedem Fall müssen diese Ergebnisdatensätze dem System aber zugeführt werden, insbesondere in Verbindung mit zugehörigen Testdatensätzen.

Das System kann dabei insbesondere auch berücksichtigen, dass es in den Testdatensätzen gegebenenfalls unterschiedliche Fehlerkategorien gibt, neben fehlerfreien Bereichen auch noch beispielsweise einen Hintergrund oder Ähnliches. In diesem Sinne können die vorbereiteten Ergebnisdatensätze beispielsweise drei Kategorien enthalten: nämlich Hintergrund, fehlerfreies Material und Fehlerstelle des Materials. Andererseits können auch mehr Klassifizierungen vorgenommen werden, indem der Unterpunkt "Fehlerstelle des Materials" weiter aufgegliedert wird in mehrere, klassifizierende Unterpunkte, wie beispielsweise "Falten", "Narben", "Warzen" oder Ähnliches. Zusätzlich oder alternativ können die Fehlerstellen auch in Qualitätsstufen eingeteilt werden, je nach Schwere des Fehlers. Dies kann das intelligente System vorteilhafterweise berücksichtigen.

So kann es beispielsweise berücksichtigen, dass bestimmte Algorithmen für bestimmte Fehlertypen (bzw. zu deren Erkennung) besonders gut geeignet sind und andere weniger.

In diesem Sinne ist beispielsweise vorstellbar, dass zunächst allgemeine Algorithmen zur Analyse verwendet werden, welche zur groben Fehlerstellenerkennung geeignet sind und dass das intelligente System anschließend andere Algorithmen an den Stellen nutzt, an welchen grundsätzlich ein Fehler aufgefunden wurde (zu deren Qualifizierung bzw. Fehlerkategorisierung).

Ein großer Vorteil der Nutzung eines intelligenten Systems, beispielsweise eines neuronalen Netzes, liegt im Sinne der vorliegenden Erfindung insbesondere darin, dass bestimmte Unregelmäßigkeiten des Körpers, wie beispielsweise Auflagefalten (einer Tierhaut oder eines Leders), zuverlässig als Nicht-Fehlerstellen erkannt werden können. Während die Systeme des Standes der Technik nicht lehrfähig sind und daher jede von der Norm abweichende Stelle, wie beispielsweise eine Auflagefalte, automatisch als Fehlerstelle einordnen, kann ein intelligentes System dazulernen und dabei auch lernen, dass bestimmte Unregelmäßigkeiten, wie beispielsweise Auflagefalten oder allgemeine Falten, gar keine Fehlerstellen darstellen. Zwar mögen diese Unregelmäßigkeiten Schattenwürfe auslösen (was zu besagter Problematik bei den Systemen des Standes der Technik führt). Tatsächlich kann die Unregelmäßigkeit aber völlig fehlerlos sein, beispielsweise für den Fall, dass sie einfach glattgezogen werden kann oder ähnliches. Ein erfindungsgemäßes intelligentes System kann, beispielsweise anhand von Testdatensätzen und entsprechenden Ergebnisdatensätzen, lernen, dass eine solche Unregelmäßigkeit (zum Beispiel eine Falte) gerade nicht als Fehlerstelle einzuordnen ist.

Hat das intelligente System dies einmal "gelernt" bzw. in einem neuronalen Netz berücksichtigt, werden derartige Falten wunschgemäß nicht mehr als Fehlerstelle angezeigt.

Unabhängig davon, ob ein intelligentes System verwendet wird oder nicht, kann die Analyse der Ausrichtungsinformationen zur Erkennung von Fehlerstellen eine Vielzahl unterschiedlicher Algorithmen verwenden. Es können beispielsweise mehr als 10, insbesondere mehr als 20, vorteilhafterweise mehr als 40, weiter sogar mehr als 50 unterschiedliche Algorithmen zur Analyse der Fehlerstellen verwendet werden bzw. in der Datenbank des Systems vorhanden sein. Wie oben beschrieben, können diese in einer besonders vorteilhaften Ausgestaltung in ein neuronales Netz eingegliedert sein.

Bei derartigen Algorithmen kann es sich beispielsweise um Waveletfilter, Gaborfilter, haraliksche Parameter, MR8-Filterbanken, local binary paterns, difference of gaussian-Algorithmen, Algorithmen beruhend auf Homogenitäts- und/oder Diskontinuitätskriterien oder Ähnliches handeln.

Die Idee der Erfindung liegt nun darin, dass diese, insbesondere an ein 3D-Verfahren angepassten, Algorithmen auf die oben beschriebenen 3D- oder - Ausrichtungdaten angewendet werden. Unabhängig davon, ob diese Algorithmen in ein neuronales Netz eingebunden sind, können diese Algorithmen zur Analyse der Ausrichtungsinformationen genutzt werden. Insbesondere kann vorgesehen sein, dass bei der Analyse eine Vielzahl von Algorithmen genutzt wird, um die Ausrichtungsinformationen zu analysieren.

Die Ausrichtungsinformationen sind dabei insbesondere Pixeln oder Kleinstbereichen zugeordnet. Wenn pro Pixel oder Kleinstbereich ein bestimmter Prozentsatz der Algorithmen eine Fehlerstelle erkennt oder voraussagt, kann erfindungsgemäß ein Fehler an besagtem Pixelort oder besagtem Kleinstbereich angenommen werden. Diese Prozentangabe ist auch als Schwellwert bezeichenbar und sie kann manuell geregelt oder eingestellt werden, beispielsweise auf einen Wert, welcher typischerweise zwischen 30 und 70 % liegt, weiter vorzugsweise bei etwa 50 %.

Gemäß einem weiteren, besonders bevorzugten Verfahren wird für jede Beleuchtungssituation genau eine der Lichtquellen angeschaltet und die anderen Lichtquellen abgeschaltet. Dies ermöglicht eine besonders genaue Rekonstruktion der Orientierungs- oder Ausrichtungsinformationen. Insofern können die Lichtquellen zeitlich nacheinander einzelnen angeschaltet werden, während die jeweils anderen Lichtquellen sämtlich ausgeschaltet sind. Dies kann von einer Rechen- oder Steuereinheit einer entsprechenden Vorrichtung erledigt werden.

Alternativ kann aber auch vorgesehen sein, dass mehrere der Lichtquellen gleichzeitig angeschaltet werden, um eine spezielle Leuchtsituation zu erreichen.

Gemäß einem besonders vorteilhaften Verfahren sind die Aufnahmeeinrichtung und die Lichtquellen innerhalb einer haubenartigen, im Wesentlichen lichtundurchlässigen Abdeckung angeordnet. Eine derartige Haube weist beispielsweise eine lichtundurchlässige Seiten- und Oberabdeckung auf. Sie kann insbesondere lediglich nach unten hin, nämlich zu einer Auflagefläche für den Körper hin, offen sein. Vorzugsweise kann der Körper unter die Haube bewegt werden (wobei auch eine genau entgegengesetzte Anordnung möglich ist, bei welcher die Haube über den Körper verfahrbar ist). Der Körper kann beispielsweise durch einen Schlitz (an der Unterseite der Abdeckung) in die Abdeckung hineinbewegt werden. Dieser Schlitz sollte derartig schmal ausgebildet werden, dass er Lichteinfall verhindert oder minimiert.

Handelt es sich bei dem Körper um eine Lederhaut kann dieser Schlitz eine Höhe (in Bezug auf die Auflagefläche des Körpers) von lediglich einigen Millimetern aufweisen, beispielsweise weniger als 50 mm, weiter insbesondere weniger als 10 mm. Der Spalt kann an der Unterseite der Abdeckung vorgesehen sein und somit eine Randöffnung darstellen oder auch vollständig in die Haube eingearbeitet sein, z.B. wenn auch die Auflagefläche durch den Schlitz hindurchreicht oder Ähnliches (in diesem Fall ist der Spalt zwischen Schlitzoberkante und Auflagefläche schmal zu halten).

Abschließend sei angemerkt, dass gemäß der Erfindung die ermittelten Fehlerstellen einem Nutzer auf einem Display einer entsprechenden Vorrichtung zur Fehlerstellenerkennung angezeigt werden können. Das Display kann beispielsweise über Leitungen oder kabellos mit einer Recheneinheit der Vorrichtung verbunden sein. Beispielsweise kann es sich um einen PC oder selbstverständlich auch um ein Tablet oder ein Handy, ein Display oder Ähnliches handeln. Alternativ können die Fehlerstellen dem Nutzer auch mittels einer Projektionseinheit der Vorrichtung derart angezeigt werden, dass die Fehlerstellen auf den Körper selber, beispielsweise auf die Haut, projiziert werden. Auf diese Weise kann ein Nutzer optisch die Güte der Fehlerstellenerkennung begutachten und gegebenenfalls mit Hilfe eines der Vorrichtung zugeordneten Eingabesystems noch nachbessern oder Ähnliches.

Von der Erfindung soll auch eine Vorrichtung gemäß dem vorliegenden Patentanspruch 10 umfasst sein, welche im Wesentlichen die Ausführung eines der beschriebenen Verfahren ermöglicht. In diesem Sinne kann eine entsprechende Vorrichtung eine Aufnahmeeinrichtung (mit beispielsweise mehreren (CCD-) Kameras) aufweisen, so wie die mehreren Lichtquellen, gegebenenfalls eine Haube und/oder eine Fördereinrichtung für den Körper. Sie kann auch eine Steuerung aufweisen, welche insbesondere eine Recheneinheit umfassen kann oder mit dieser verbunden sein kann, die die benötigte Rechenleistung zur Durchführung des oben beschriebenen Verfahrens bereitstellt.

Weitere Vorteile der vorliegenden Erfindung ergeben sich anhand gegebenenfalls nicht zitierter Unteransprüche sowie anhand der nachfolgenden Figurenbeschreibung.

Darin zeigen:
- Fig. 1: in einer sehr schematischen, perspektivischen, teiltransparenten Ansicht eine Darstellung einer Vorrichtung zur Durchführung des erfindungsgemäßen Verfahrens bei aufgelegtem, bereits positioniertem Körper im Bereich einer optischen Haube,
- Fig. 2: ein Ablaufdiagramm für ein Ausführungsbeispiel des erfindungsgemäßen Verfahrens,
- Fig. 3a: in einer ganz schematischen, vergrößerten Ansicht ein Ausschnitt aus dem Bereich des Körpers gemäß Fig. 1 mit einer sehr kleinen, exemplarischen Fehlerstelle unter teilweiser Darstellung von Flächennormalen,
- Fig. 3b: eine sehr schematische Schrägdarstellung einer Abbildung des in Fig. 3a dargestellten Bereiches, unter Hinzufügung exemplarischer Normalen für die einzelnen Kleinstbereiche bzw. Pixel,
- Fig. 4: ein sehr exemplarisches und schematisches Ablaufdiagramm eines Trainings des im erfindungsgemäßen Verfahren eingesetzten neuronalen Netzes, und
- Fig. 5: in einer sehr schematischen Aufsicht oder Displayansicht eine Darstellung des Körpers, wie er in abweichender Dimensionierung aus Fig. 1 bekannt ist, wobei Fehlerstellen innerhalb des Körpers dargestellt sind, sowie Projektionen von im Rahmen eines Nesting-Prozesses verteilter Zuschnittteile.

Der folgenden Figurenbeschreibung sei vorangestellt, dass gleiche oder vergleichbare Teile mit identischen Bezugszeichen versehen sein können, teilweise unter der Hinzufügung kleiner Buchstaben oder von Apostrophs, wobei diese in den der Figurenbeschreibung nachfolgenden Patentansprüchen der Übersichtlichkeit halber gegebenenfalls fortgelassen sind.

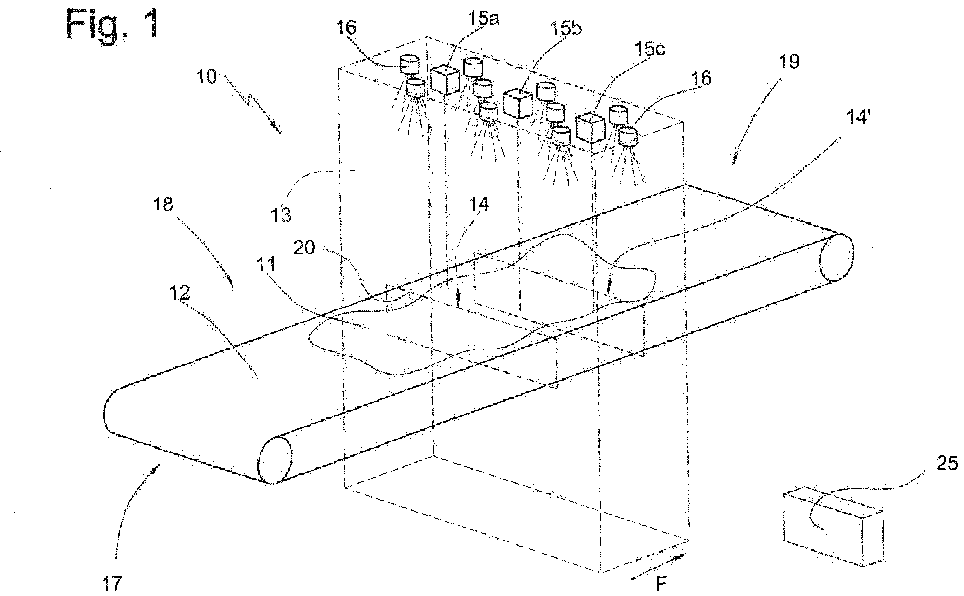

Fig. 1 zeigt zunächst eine Vorrichtung 10 zur Durchführung eines erfindungsgemäßen Verfahrens. Die Vorrichtung 10 ermöglicht dabei die Fehlerstellenerkennung eines biegeschlaffen Körpers 11, welcher in dem vorliegenden Ausführungsbeispiel beispielsweise als Leder- oder Tierhaut ausgebildet ist.

Das Leder oder der Körper 11 ist hierbei auf eine Auflagefläche 12 aufgelegt, welche in dem vorliegenden Ausführungsbeispiel exemplarisch einer Fördereinrichtung 17 nach Art eines umlaufenden Förderbandes zugeordnet ist. Ein Nutzer kann den biegeschlaffen Körper 11, beispielsweise in einem Auflagebereich 18 der Fördereinrichtung 17, auf der Auflagefläche 12 positionieren, und die Auflageeinrichtung 17 kann den Körper 11 dann abschnittsweise in den Bereich einer Abdeckung oder Haube 13 transportieren.

Die Abdeckung 13 dient dabei der Abschirmung des (im Bereich der Haube 13 befindlichen Bereiches des) Körpers 11 vor Außenlicht. Die Haube oder Abdeckung 13 ist in Fig. 1 der Übersichtlichkeit halber gestrichelt bzw. transparent dargestellt, um den Blick auf den Körper 11 sowie auf innerhalb der Abdeckung angeordnete Kameras 15a, 15b, 15c und Lichtquellen 16 nicht zu versperren (die Haube 13 ist tatsächlich aber gerade nicht transparent).

Der Körper 11 kann in den Bereich der Haube 13 durch einen Eintrittsspalt 14 eintreten und - in dem vorliegenden Ausführungsbeispiel - die Haube 13 durch einen bezüglich der Förderrichtung F nachfolgenden Austrittsspalt 14' auch wieder verlassen. Durch den Austrittsspalt 14' kann der Körper 11 nach Durchführung des erfindungsgemäßen Verfahrens in einen Entnahme- oder Projektionsbereich 19 gelangen.

In einem gleichwertigen, nicht dargestellten Ausführungsbeispiel kann auf einen separaten Entnahmenbereich 19 aber auch verzichtet werden. So kann der Körper 11 zwecks Entnahme in besagtem Ausführungsbeispiel beispielsweise gegen die Förderrichtung F in den Auflagebereich 18 zurückverfahren (nach der Durchführung des erfindungsgemäßen Verfahrens) und dort entnommen werden (in diesem Fall kann ein Austrittsspalt 14' entfallen).

Der Spalt 14 bzw. die Spalte 14 und 14' sind dabei derart gewählt, dass möglichst kein Außenlicht durch diese ins Innere der Abdeckung 13 gelangt. In dem in Fig. 1 dargestellten Ausführungsbeispiel werden die Spalte 14 und 14' im Wesentlichen von dem Querschnitt der Fördereinrichtung 17 ausgefüllt. In einem anderen, nicht dargestellten Ausführungsbeispiel, bei welchem die Abdeckung 13 nicht auf den Bodenbereich reicht, sondern lediglich in den Bereich bis zur Auflagefläche 12, kann der Spalt auch an oder durch die Unterseite der Abdeckung 13 gebildet werden. Wichtig ist hierbei, dass die obere Spaltkante 20 einen möglichst geringen Abstand zur Auflage12 bzw. zum Körper 11 aufweist. Dieser Abstand ist bei der erfindungsgemäßen Vorrichtung minimiert.

Von entscheidender Bedeutung ist bei dem Ausführungsbeispiel gemäß Fig. 1, dass im Inneren der Abdeckung 13, nämlich beispielsweise in deren Deckenbereich, eine Aufnahmeeinrichtung 15 angeordnet ist. Diese Aufnahmeeinrichtung 15 besteht in dem vorliegenden Ausführungsbeispiel exemplarisch aus drei Digitalkameras 15a, 15b, 15c. Diese sind in dem Ausführungsbeispiel auf einer Geraden quer zur Förderrichtung F angeordnet und mit ihrem Blickfeld jeweils nach unten, zum Körper 11 hin, ausgerichtet (diese Ausrichtung wird in Fig. 1 exemplarisch durch senkrechte, gestrichelte Linien angedeutet, welche aber nicht den Aufnahmebereich andeuten, sondern lediglich die grundsätzliche Ausrichtung der Kameras 15a, 15b , 15c erklären sollen).

Die Kameras 15a, 15b, 15c bzw. die Aufnahmeeinrichtung 15 können beispielsweise im Deckenbereich der Haube 13 fest angeordnet sein oder an einem separaten Gestell im Inneren der Haube oder Ähnliches.

Gleiches gilt für die Befestigung von in Fig. 1 dargestellten, ebenfalls im Inneren der Haube 13 angeordneten Lichtquellen 16. Bei den Lichtquellen 16 handelt es sich im Wesentlichen um punktartige Lichtquellen (beispielsweise LED-Lichtquellen). In Fig. 1 ist die Anordnung der Lichtquellen derart gewählt, dass jede der Kameras 15a, 15b, 15c gleich beabstandet von vier Lichtquellen 16 umgeben ist. Daher sind in Fig. 1 exemplarisch genau 10 Lichtquellen vorhanden.

Auch wenn es sich bei den Lichtquellen 16 grundsätzlich um Punktlichtquellen handelt, können diese in dem Ausführungsbeispiel insbesondere auch eine Abschirmung nach oben hin vorsehen, so dass es im Deckenbereich der Haube 13 nicht zu zuviel Reflektionen kommt.

Bezüglich Fig. 1 sei angemerkt, dass die Höhe der Haube 13 oberhalb der Auflagefläche 12 derart gewählt ist, dass die Aufnahmeeinrichtung 15, bzw. die einzelnen Kameras 15a, 15b, 15c, die Oberseite des Körpers 11 fokussiert abbilden können. Wäre die Höhe der Haube 13 zu niedrig gewählt, wäre dies gegebenenfalls nicht möglich (der Körper 11 könnte gegebenenfalls nicht scharf abgebildet werden). Je nach Dimensionierung der Haube 13 (bzw. nach Dimensionierung des Körpers 11) kann der Körper 11 gegebenenfalls ganz in den Bereich der Haube 13 eingefahren (uns somit auch von der Aufnahmeeinrichtung mit einer einzigen Aufnahme in Gänze erfasst) werden oder alternativ - wie in dem vorliegenden Ausführungsbeispiel - segment- oder abschnittsweise in den Bereich der Haube 13 bzw. in den Bereich der Aufnahmeeinrichtung 15 eingefahren werden.

In dem vorliegenden Ausführungsbeispiel gemäß Fig. 1 sei exemplarisch angegeben, dass der aufnehmbare/abbildbare Bereich der Oberfläche 12 bzw. des Körpers 11 eine Dimensionierung aufweist von etwa 3 m quer zur Förderrichtung F und etwa 80 cm in Förderrichtung F. Der abbildbare Bereich kann also eine Breite von etwa 3 m, vorzugsweise von zwischen 1 und 5 m aufweisen, bei einer Länge von etwa 80 cm, vorzugsweise von zwischen 50 cm und 3 m. Im Bereich der Haube 13 sind exemplarisch drei Kameras 15a, 15b, 15c angeordnet. Bei anderen Haubendimensionierungen ist natürlich auch der Einsatz von weniger oder mehr Kameras möglich. Sind mehr Kameras 15 vorhanden, können diese selbstverständlich entlang derselben Geraden angeordnet werden oder sogar gitter- oder rasterartig im oberen Haubenbereich verteilt.

Soviel zu der grundsätzlichen Ausgestaltung der Vorrichtung 10 gemäß Fig. 1, mit welcher die Durchführung eines erfindungsgemäßen Verfahrens möglich ist.

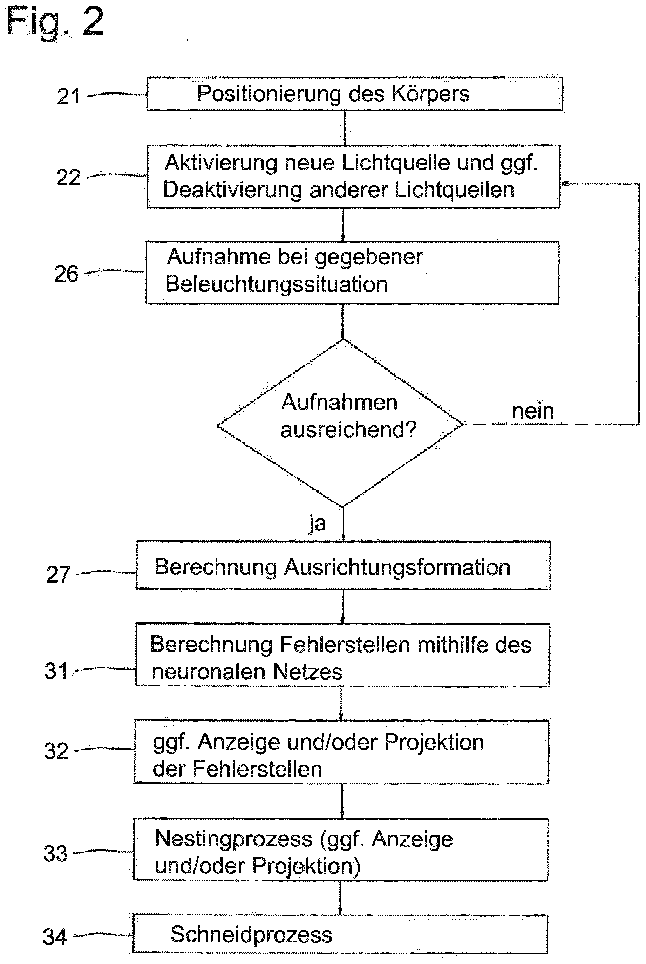

Der Ablauf eines derartigen erfindungsgemäßen Verfahrens zur Fehlerstellenerkennung wird im Ablaufdiagramm gemäß Fig. 2 exemplarisch dargestellt und nunmehr erörtert:
So wird in einem Verfahrensschritt 21 der Körper 11 positioniert, also beispielsweise zunächst auf der Auflage 12 im Auflagebereich 18 der Fördereinrichtung 17 aufgelegt. Der Körper 11 kann anschließend in die Haube 13 ein- oder teileingefahren werden, was ebenfalls noch zum Schritt 21 gehört.

Anschließend kann typischerweise genau eine der Lichtquellen 16 in einem Arbeitsschritt 22 aktiviert werden. Hierbei ist, wie beschrieben, vorzugsweise lediglich eine der Lichtquellen 16 aktiv bzw. angesteuert, während die restlichen Lichtquellen abgeschaltet sind/werden.

Die Ansteuerung bzw. Aktivierung oder Deaktivierung der Lichtquellen 16 kann mit Hilfe einer in Fig. 1 lediglich angedeuteten Recheneinheit 25 erfolgen. Diese Recheneinheit 25 kann mit den Lichtquellen 16 kabellos oder über nicht dargestellte Kabel oder Leitungen verbunden sein. Die Recheneinheit 25 ermöglicht somit die Ansteuerung der Lichtquellen 16. Gemäß Schritt 22 werden insbesondere sämtliche anderen Lichtquellen 16 als die ausgewählte, aktuelle Lichtquelle 16 abgestellt (in Fig. 1 sind lediglich verständnishalber sämtliche Lichtquellen 16 gleichzeitig aktiv).

Nachdem die einzelne Lichtquelle 16 angeschaltet und die anderen Lichtquellen 16 abgeschaltet sind, wurde somit eine neue Beleuchtungssituation geschaffen, bei welcher anschließend, nämlich gemäß Schritt 26, eine Aufnahme durch die Aufnahmeeinrichtung 15 erfolgen kann.

Hierbei tätigen typischerweise sämtliche Kameras 15a, 15b, 15c der Aufnahmeeinrichtung 15 gleichzeitig jeweils eine (Teil-) Aufnahme, nämlich bei der soeben beschriebenen Beleuchtungssituation. In diesem Sinne erstellt jede der Kameras 15a, 15b 15c eine Teilaufnahme der Gesamtaufnahme, welche von der Aufnahmeeinrichtung 15 erstellt wird. Die Gesamtaufnahme der Aufnahmeeinrichtung 15 setzt sich somit aus mehreren Teilbildern zusammen, welche von den Teilaufnahmen der Kameras 15a, 15b, 15c bereitgestellt werden. Die Recheneinheit 25 kann die Teilaufnahmen der Kameras 15a, 15b, 15c hierzu nach in der Fotografie- oder Bildbearbeitung bekannten Mitteln zusammensetzen, beispielsweise indem überlappende Bereiche herausgeschnitten oder herausgerechnet werden.

Somit entsteht beim Arbeitsschritt 26 eine vollständige Aufnahme des Teilbereiches des Körpers 11, welcher sich im Bereich der Abdeckung 13 befindet.

Aus einer derartigen Aufnahme bei einer gegebenen Beleuchtungssituation lassen sich allerdings noch keine Ausrichtungsinformationen für Kleinstabschnitte des Körpers 11 berechnen. Hierzu bedarf es mehrerer Aufnahmen bei unterschiedlichen Beleuchtungssituationen.

Daher wird anschließend, insbesondere bei gleicher Positionierung des Körpers 11, eine andere Beleuchtungssituation geschaffen, nämlich wieder gemäß dem Arbeitsschritt 22.

Nunmehr wird vielmehr aber eine andere Lichtquelle 16 aktiviert und die vorher aktive Lichtquelle wird deaktiviert. Hierdurch ist eine zweite Beleuchtungssituation entstanden, in welcher in einem weiteren Arbeitsschritt 26 wieder eine neue, zweite Aufnahme nach oben beschriebener Art erstellt wird.

Auf diese Weise kann eine Vielzahl von Aufnahmen entstehen, welche jeweils bei einer anderen, unterschiedlichen Beleuchtungssituation getätigt wurden.

In dem Ausführungsbeispiel können beispielsweise 10 Aufnahmen bei unterschiedlichen Beleuchtungssituationen getätigt werden. Hierbei gilt: Je mehr Aufnahmen bei unterschiedlichen Beleuchtungssituationen erstellt werden, desto besser ist tendenziell die Berechnung der Ausrichtungsinformationen (wobei auch eine Überbestimmung erreicht werden kann).

Mindestens sollten aber drei, vorzugsweise vier oder mehr, Aufnahmen getätigt werden. Ist die vorgegebene Anzahl von Aufnahmen bei unterschiedlichen Beleuchtungssituationen erreicht, können die Ausrichtungsinformationen für Kleinstbereiche auf dem Körper 11 gemäß Schritt 27 berechnet werden. Dies wird in der Regel ebenfalls von einer Recheneinheit durchgeführt, vorzugsweise von derselben Recheneinheit 25. Diese Recheneinheit 25 kann die Aufnahmen hierzu jeweils identisch in mehrere Kleinstbereiche, beispielsweise Pixel, unterteilen. Jedem Pixel der mehreren Aufnahmen kann nunmehr eine Ausrichtungsinformation zugeordnet (bzw. diese Ausrichtungsinformation berechnet) werden, welche die geometrische Ausrichtung des Abschnittes des Körpers 11 beschreibt, der dem Kleinstbereich bzw. Pixel in der Aufnahme entspricht.

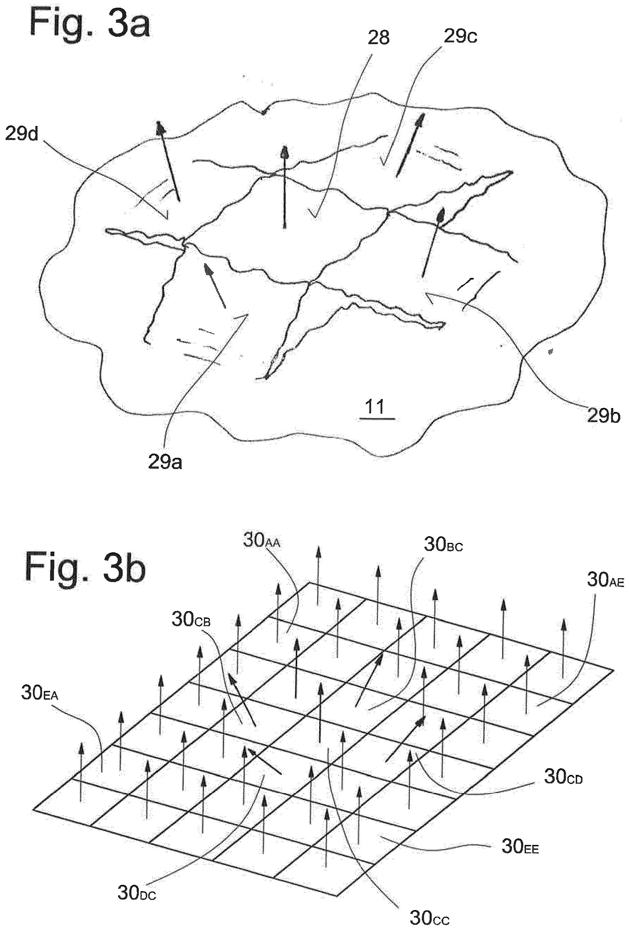

Zu einem besseren Verständnis wird diesbezüglich exemplarisch auf Fig. 3 verwiesen:
Fig. 3a zeigt hierbei sehr exemplarisch einen sehr kleinen Ausschnitt aus der Oberseite des Körpers 11. Dieser Körper 11 weist offensichtlich eine Fehlerstelle 23 nach Art eines rechteckigen Domes auf. Eine solche rechteckige Fehlerstelle wird in der Praxis oder Natur tatsächlich nie vorkommen. Sie ist aber besonders gut zur Visualisierung oder Verdeutlichung des erfindungsgemäßen Verfahrens geeignet und sei daher rein exemplarisch gewählt. Die Fehlerstelle 23 weist, wie beschrieben, eine rechteckige Domstruktur auf mit einem oberen Plateau 28 sowie von diesem eckig abfallenden, im Wesentlichen rechteckigen, symmetrischen Flankenbereichen 29a, 29b, 29c und 29d.

Wird von dem Bereich des Körpers 11 gemäß Fig. 3a eine Aufnahme getätigt, so erstreckt sich diese etwa über einen Bereich, wie er in Fig. 3b dargestellt ist. Hier ist der in Fig. 3a dargestellte Ausschnitt in mehrere Kleinstbereiche oder Pixel 30 eingeteilt. Der Bereich weist exemplarisch jeweils fünf Pixelspalten und fünf Pixelzeilen auf.

Es handelt sich bei einer Kameraaufnahme naturgemäß grundsätzlich um eine zweidimensionale Abbildung. In dem vorliegenden Ausführungsbeispiel gemäß Fig. 3b handelt es sich bei den Kleinstbereichen 30 also um Pixel (ein Kleinstbereich kann in einem anderen Ausführungsbeispiel aber auch mehrere Pixel umfassen).

Im Arbeitsschritt 27 gemäß Fig. 2 werden nun für jeden in Fig. 3b dargestellten Kleinstbereich oder Pixel 30 Ausrichtungsinformationen nach Art eines Normalenvektors berechnet. Dies geschieht auf Basis der oben beschriebenen Arbeitsschritte (insbesondere 22 und 26), also durch Auswertung der mehreren Aufnahmen bei unterschiedlichen Beleuchtungssituationen.

Fig. 3b lässt sich hierbei entnehmen, dass den Außenbereichen des dargestellten Körperausschnittes Normalenvektoren zugeordnet sind, welche gleichzeitig Normalen der in Fig. 3b dargestellten flachen Hauptebene ausbilden. Dies gilt beispielsweise für die oberen Pixel 30_{AA} bis 30_{AE}.

Da aber auch das Domplateau 28 gemäß Fig. 3a (welches in der Abbildung zufällig die Dimensionierung etwa eines Pixels aufweist) flach ausgebildet ist, weist auch das Pixel 30_{CC} einen identischen Normalenvektor auf, der normal zur aufgespannten Hauptebene ausgerichtet ist.

Anderes gilt selbstverständlich für die Flankenbereiche 29a, 29b, 29c und 29d gemäß Fig. 3a. So zeigt Fig. 3b für die entsprechenden Pixel 30_{DC},30_{CD}, 30_{BC} und 30_{CB} gerade abweichende Normalenvektoren. Diese repräsentieren die Ausrichtung der Flankenbereiche 29 in Fig.3a. Hierbei kann es sich typischerweise um die durchschnittlichen Normalen handeln, da die Flankenbereiche 29, wie eigentlich sämtliche Kleinstbereiche auf dem Körper 11, natürlich naturgemäß nicht exakt als ebene Rechtecke oder Quadrate ausgebildet sind.

Die Normalenvektoren, welche in Fig. 3b dargestellt sind, dienen lediglich der Visualisierung.

Tatsächlich wird im Rahmen des erfindungsgemäßen Verfahrens bevorzugt jedem Kleinstbereich oder Pixel 30 kein optischer Vektor zugeordnet, sondern ein Vektor nach Art von Zahlenwerten, beispielsweise ein 3-Tupel an Werten. Auf diese Weise wird für sämtliche Kleinstbereiche oder Pixel des abgebildeten Bereiches auf Basis der mehreren Aufnahmen eine Karte des Aufnahmebereiches erstellt, bei welcher jedem Kleinstbereich eine entsprechende Ausrichtungsinformation (ein n-Tupel an Werten) zugeordnet ist.

In einem ebenfalls umfassten, aber nicht visualisierten Verfahrensschritt kann eine solche Karte (im Englischen "normal map" genannt) auch visualisiert und dem Benutzer angezeigt werden. In der Praxis wird dabei eine Visualisierung nach Art einer 2D-Karte mit Farbbereichen im Blau-, Gelb-, Magenta-, Cyan-, Grün- und Rot-Bereich erstellt: So zeigen sämtliche Bereiche, in welcher die Normale einer Fläche zur Aufnahmeeinrichtung hinzeigt, typischerweise eine blaue Farbgebung, hiervon abweichende je nach Richtung eine andere Farbe.

Jedenfalls kann durch Auswertung dieser Ausrichtungsinformationen, (beispielsweise nach Art einer "normal map") nunmehr die Berechnung der Fehlerstellen des aufgenommenen Bereiches in einem Arbeitsschritt 31 erfolgen.

Hierzu wird typischerweise ein aus mehreren Algorithmen bestehendes neuronales Netz eingesetzt, wie es später noch genauer beschrieben wird.

Wurde auf diese später noch genauer beschriebene Art und Weise die Berechnung von Fehlerstellen durchgeführt, können diese Fehlerstellen in einem optionalen Arbeitsschritt 32 auch noch angezeigt und/oder auf den Körper projiziert werden. Die Anzeige kann dabei beispielsweise auf einem Display außerhalb der Haube 13 erfolgen. Soll eine Projektion der Fehlerstellen in diesem Arbeitsschritt 32 erfolgen, so würde der aufgenommene Bereich des Körpers 11, welcher vollständig in die Haube eingefahren wurde, typischerweise wieder aus der Haube heraustransportiert werden. Die Vorrichtung 10 kann dafür eine nicht dargestellte Projektionseinheit umfassen, welche die Fehlerstellen auf den Körper projiziert. Ein Nutzer kann hierbei dann manuell überprüfen, ob die durchgeführte Fehlerstellenerkennung die Fehlerstellen hinreichend erkannt hat (anderenfalls kann gegebenenfalls ein Rücksprung zur Position 21 erfolgen).

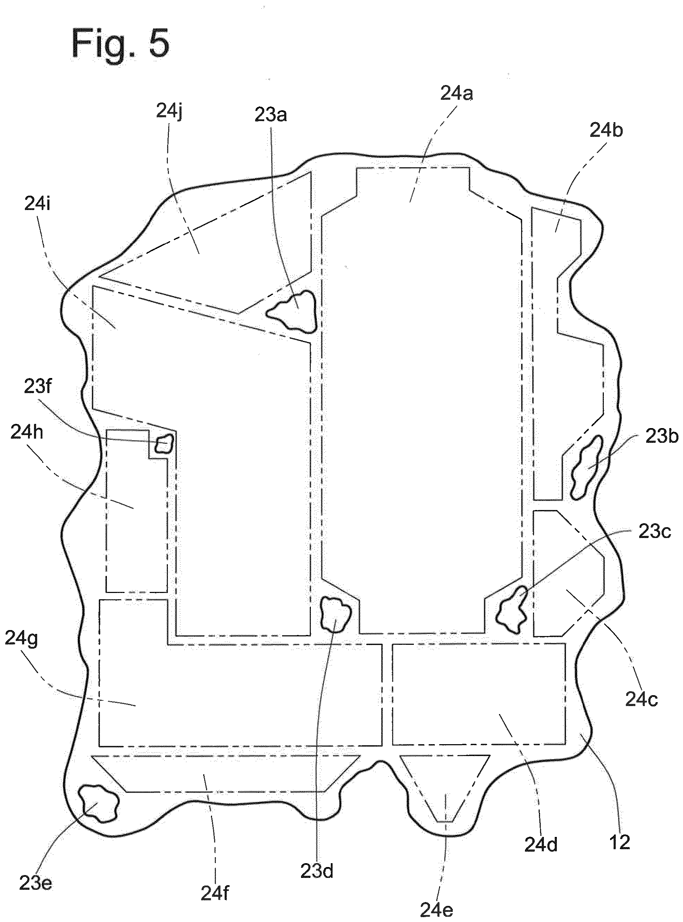

Wurde der Arbeitsschritt 32 ausgelassen oder wurde das angezeigte Ergebnis als befriedigend angesehen, kann in einem weiteren Arbeitsschritt 33 ein sogenannter Nestingprozess erfolgen. Dieser wird typischerweise ebenfalls durch die Recheneinheit 25 oder durch eine weitere, andere durchgeführt. Dieser Nesting-Schritt kann dabei die ermittelten Fehlerstellen bei der Ausrichtung und Zuteilung von aus dem Körper 11 auszuschneidenden Stücken 24 berücksichtigen, wie dies rein exemplarisch in Fig. 5 dargestellt ist. Anhand der detektierten bzw. berechneten Fehlerstellen 23a, 23b, 23c, 23d, 23e, 23f kann die Recheneinheit 25 besagten herkömmlichen Nestingprozess durchführen und auszuschneidende Bearbeitungsstücke 24 derart auf der Haut verteilen, dass die erkannten Fehlerstellen 23 nicht innerhalb der auszuschneidenden Stücke 24 liegen.

Die Umrisse der verteilten bzw. auszuschneidenden Stücke 24 können an besagtem, nicht dargestellten Display zwecks optischer Kontrolle (erneut) dargestellt werden. Alternativ können die Konturen dieser Ausschnittstücke 24 auch, wie bezüglich des Arbeitsschrittes 32 bereits erläutert, durch die nicht dargestellten Projektionseinheit, zwecks optischer Kontrolle, auf herkömlich bekannte Art auf den Körper 11 projiziert werden.

Abschließend kann in einem finalen Arbeitsschritt die Zerteilung des Körpers 11 erfolgen, beispielsweise mit Hilfe eines Schneidprozesses 34 oder mit Hilfe eines Stanzprozesses oder eines sonstigen Teilungsprozesses oder Ähnliches, welcher besagten Nestingprozess gemäß Fig. 5 berücksichtigt. Dieser Schneidprozess kann dabei typischerweise an einem gesonderten Schneidtisch erfolgen, welcher aber ebenfalls mit der Recheneinheit 25 verbunden sein kann. Insbesondere kann diese den Schneidprozess auf Basis der im Arbeitsschritt 33 ermittelten Informationen steuern oder Ähnliches. Gegebenenfalls können die Ergebnisse des Nestingprozesses 33 auch zunächst gespeichert und später für den Schneidprozess 34 verwendet werden.

Bezüglich des Ablaufdiagramms gemäß Fig. 2 sei abschließend angemerkt, dass dieses die Fehlerstellenerkennung für den Fall beschreibt, dass entweder der gesamte Körper 11 als Ganzes in dem aufnehmbaren Bereich der Haube 13 positioniert wird oder das lediglich ein Teilbereich des Körpers 11 hinsichtlich seiner Fehler analysiert werden soll. Sollen sämtliche Bereiche des Körpers, welche nicht gleichzeitig in die Haube 13 passen, hinsichtlich von Fehlerstellen analysiert werden, so erfolgt dies typischerweise segmentiert, derart, dass zunächst ein erster Teil des Körpers 11 in den Bereich der Haube 13 eingefahren wird oder dort positioniert wird, entsprechende Aufnahmen getätigt werden, (gegebenenfalls bereits Berechnungen erstellt werden) und dann ein zweiter Bereich des Körpers 11 in die Haube 13 eingefahren oder dort positioniert wird, wobei dann separate Aufnahmen und/oder Berechnungen erfolgen. Dies wird für so viele Abschnitte des Körpers 11 wie nötig wiederholt. Dabei oder anschließend können entweder die Aufnahmen oder die Ausrichtungsinformationen oder bereits die Fehlerstelleninformationen zu Informationsbündeln für den ganzen Körper 11 zusammengesetzt werden.

Gemäß einem weiteren Aspekt sei darauf hingewiesen, dass die Berechnung der Fehlerstellen im Arbeitsschritt 31 gemäß Fig. 2 in dem vorliegenden Ausführungsbeispiel mit Hilfe eines neuronalen Netzes geschieht. Der Vollständigkeit halber sei angemerkt, dass die Erfindung hierauf grundsätzlich aber nicht beschränkt ist.

In einem anderen, nicht visualisierten Ausführungsbeispiel könnten die Ausrichtungsinformationen sehr wohl auch ohne Zuhilfenahme eines neuronalen Netzes berechnet werden, indem beispielsweise herkömmliche, z.B. auf Nachbarschaftsbeziehungen beruhende Algorithmen die vorhandenen Informationen - erfindungsgemäß nämlich Ausrichtungsinformationen - analysieren. Anstelle der aus dem Stand der Technik bekannten Helligkeits- oder Farbwerte können hierbei dann allerdings die Ausrichtungsinformationen verwertet werden. Da es sich hierbei typischerweise um 3D-Werte handelt, werden in der Regel modifizierte Algorithmen eingesetzt, welche vorzugsweise im dreidimensionalen Raum arbeiten.

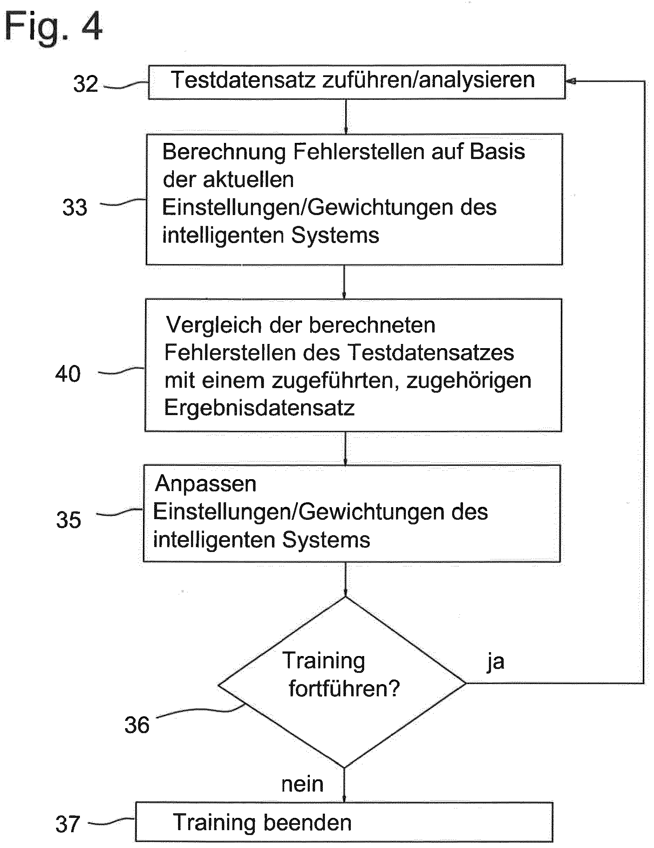

In dem in Fig. 2 dargestellten Ausführungsbeispiel erfolgt diese Analyse aber mit Hilfe eines intelligenten Systems, insbesondere eines neuronalen Netzes. Ein derartiges neuronales Netz wird typischerweise vor dem Einsatz bei einem Nutzer der beschriebenen Vorrichtung zunächst (werksseitig) trainiert. Wie ein derartiges Training erfolgen kann, zeigt der Ablauf gemäß Fig. 4. Dem System wird hierbei nämlich zunächst ein erster Testdatensatz zugeführt. Bei dem Testdatensatz kann es sich typischerweise um eine bereits vorbereitete oder berechnete Karte mit Ausrichtungsinformationen (beispielsweise eine "normal map") handeln. Zu einem derartigen Testdatensatz besteht bereits ein zugehöriger Ergebnisdatensatz, welcher Informationen über die tatsächlich enthaltenen Fehlerstellen aufweist. So kann ein Ergebnisdatensatz beispielsweise manuell durch rein optisch-manuelle Beobachtungen erstellt werden oder mit Hilfe eines anderen Systems vorbereitet werden.

Alternativ könnte der Testdatensatz grundsätzlich aber auch in Form mehrerer Aufnahmen eines Körpers vorliegen, aus welchen das System dann zunächst die Ausrichtungsinformationen ermitteln würde. Schließlich ist sogar vorstellbar, dass anstelle eines Testdatensatzes dem System ein biegeschlaffer Körper zugeführt wird, zu welchem ein Ergebnisdatensatz bereits bekannt ist. Der Testdatensatz kann in diesem Fall von dem System selber erstellt werden. Das System kann sich den Testdatensatz dann selber "zuführen".

Typischerweise erfolgt das Training aber ausgehend von dem erstgenannten Schritt, nach welchem der Testdatensatz bereits Ausrichtungsinformationen nach Art einer "normal map" enthält.

Dieser Schritt des Zuführens eines Testdatensatzes ist in Fig. 4 mit der Bezugsziffer 38 gekennzeichnet.

Gemäß Arbeitsschritt 39 ermittelt das intelligente System dann Fehlerstellen auf Basis seiner aktuellen Einstellungen und/oder Gewichtungen. Bei den aktuellen Einstellungen des Systems handelt es sich am Anfang demnach um initiale Einstellungen, welche beispielsweise werksseitig vorgegeben werden können oder welche sich das System zufällig wählt.

Hierbei kann es sich beispielsweise um Gewichtungen einzelner Algorithmen handeln. So kann beispielsweise einem ersten Algorithmus mehr Einfluss für das Ergebnis zugestanden werden als einem zweiten Algorithmus. Dies wird in dem Fall wichtig, dass beide Algorithmen auf unterschiedliche Ergebnisse kommen.

Typischerweise durchlaufen beim Arbeitsschritt 39 zahlreiche Algorithmen des intelligenten Systems eine Fehlerstellenerkennung und kommen hierbei gegebenenfalls zu unterschiedlichen Ergebnissen (für die einzelnen Kleinstbereiche/Pixel).

Ergebnisse können beispielsweise in einer Aussage darüber liegen, ob einem bestimmten Kleinstbereich oder einem Pixel eines Datensatzes eine Fehlerstelle zugeordnet ist. Grundsätzlich können auch weitere Ergebnisse vorgesehen sein: Nämlich neben dem Vorhandensein und Nicht-Vorhandensein einer Fehlerstelle beispielsweise auch eine Aussage dahingehend, dass es sich weder um einen fehlerfreien noch um einen fehlerbehafteten Bereich des Körpers handelt sondern um einen reinen Hintergrundbereich, wie beispielsweise die Auflage 12.

Die einzelnen Algorithmen können bei ihrer Berechnung zu unterschiedlichen Ergebnissen und/oder Wahrscheinlichkeiten kommen.

In einem Arbeitsschritt 40 kann das entsprechende Ergebnis dann mit dem ebenfalls bereitgestellten zugehörigen Ergebnisdatensatz verglichen werden. Dieser Abgleich lässt dann Rückschlüsse darüber zu, welche Algorithmen sich zur Erkennung, ob tatsächlich ein Fehler vorliegt, als besonders geeignet erweisen.

Vorzugsweise kann der Ergebnisdatensatz auch Aussagen über die Qualifizierung einer Fehlerstelle enthalten. Auf diese Weise kann dann von dem intelligenten System eingeschätzt werden, welche Algorithmen zur Erkennung welcher Art von Fehlerstellen besonders geeignet sind.

In einem weiteren Arbeitsschritt 35 kann das intelligente System die oben genannten Einstellungen und/oder Gewichtungen dann nach den gewonnenen Erkenntnissen (durch den Abgleich gemäß Arbeitsschritt 40) anpassen. Einige Algorithmen, welche sich als besonders zuverlässig erweisen, können beispielsweise stärker gewichtet werden oder Ähnliches.

Anschließend kann ein weiterer Testdatensatz zugeführt oder ein weiterer bereitgestellter Testdatensatz analysiert werden. Hat sich bei 36 dieser Ablauf hinreichend oft wiederholt (welche Anzahl beispielsweise werkseitig einstellbar ist), kann das Training gemäß Punkt 37 alternativ auch beendet werden. Das intelligente System hat sich nunmehr selber kalibriert.

Wie oben bereits geschildert, erfolgt dieser Trainingsablauf gemäß Fig. 4 typischerweise werksseitig. Auf Kundenwunsch kann die Kalibrierung auch durch den Kunden erfolgen oder erweitert werden (mit Hilfe von mehr Datensätzen). Auf diese Weise lernt das System stets weiter.

Andererseits besteht auch die Möglichkeit, dass nach Abschluss des werkseitigen Trainings dem Benutzer keine Einstellungen mehr zur Verfügung gestellt werden, das System weiter zu trainieren. Das System würde in diesem Fall, obwohl es sich um ein neuronales Netz handelt, dann auch beim Kunden nicht weiterlernen, wenn dies gewünscht ist.

Alternativ kann das System auch beim Kunden weitertrainiert werden, auch nicht nur mit Hilfe von Testdatensätzen, sondern in realen Anwendungsfällen: In diesen Fällen ist dann aber abschließend von dem Kunden oder Nutzer eine Bewertung des berechneten Ergebnisses abzugeben, welches dann gemäß Arbeitsschritt 40 den Vergleich mit einem (manuell beobachteten) Ergebnisdatensatz ersetzt.

Bei den verwendeten Algorithmen kann es sich beispielsweise um die oben bereits genannten Algorithmen wie Wavelet-Filter, Gabor-Filter, local binary paterns, Difference of Gaussion-Methoden, haraklische Parameter oder Ähnliches handeln. Jeder dieser Algorithmen untersucht dabei den Testdatensatz bzw. im realen Anwendungsfall die Aufnahmen grundsätzlich separat voneinander.

Wird kein intelligentes System verwendet, können diese Algorithmen gleichgewertet sein oder werksseitig einmalig gewertet werden. Für eine Gleichwertung sei als Beispiel angeführt, dass, wenn z.B. 50 % der angewendeten Algorithmen einen Pixel oder Kleinstbereich als fehlerbelastet berechnen, dieser Bereich als fehlerhaltig markiert wird. Diese Schwelle kann werkseitig oder nutzerseitig einstellbar bzw. regelbar sein.

## Patentansprüche

1. Verfahren zur Fehlerstellenerkennung bei biegeschlaffen Körpern (11), insbesondere bei Tierhäuten, umfassend die Schritte
- Bereitstellen einer oder mehrerer, insbesondere von mindestens drei, weiter insbesondere von mindestens fünf, Lichtquelle(n) (16), vorzugsweise Punktlichtquelle(n), zur Beleuchtung mindestens eines Teilbereichs des Körpers (11),
**gekennzeichnet durch** folgende Schritte:
- Erstellen mehrerer Aufnahmen mindestens des Teilbereichs des Körpers (11) durch eine, insbesondere mindestens eine Kamera (15a, b, c) aufweisende, Aufnahmeeinrichtung (15),
- wobei jede der mehreren Aufnahmen bei einer anderen von der/den Lichtquelle(n) (16) erzeugten Beleuchtungssituation erfolgt, insbesondere derart, dass für jede Aufnahme eine andere der mehreren Lichtquellen (16) aktiviert wird,
- Berechnung von Ausrichtungsinformationen, insbesondere dem Normalenvektor, von dem Körper (11) zugeordneten Kleinstbereichen (30), beispielsweise Pixeln, anhand der mehreren Aufnahmen,
- Analyse der Ausrichtungsinformationen zur Erkennung von Fehlerstellen des Körpers (11).

2. Verfahren nach Anspruch 1, **dadurch gekennzeichnet, dass** bei Berechnung der Ausrichtungsinformationen die Position(en) der Lichtquelle(n) (16) sowie der Aufnahmeeinrichtung (15) und/oder Helligkeits- oder Farbwerte der Kleinstbereiche (30) In den mehreren Aufnahmen berücksichtigt werden, insbesondere nach Art eines photometrischen Stereoverfahrens.

3. Verfahren nach Anspruch 1 oder 2, **dadurch gekennzeichnet, dass** die Analyse der Ausrichtungsinformationen zur Erkennung der Fehlerstellen des Körpers (11) durch ein intelligentes System, insbesondere nach Art eines neuronalen Netzes, weiter insbesondere nach Art eines convolutional neuronalen Netzes, erfolgt.

4. Verfahren nach Anspruch 3, **dadurch gekennzeichnet, dass** das intelligente System trainiert wird, indem Testdatensätze und zugehörige Ergebnisdatensätze bereitgestellt werden, wobei ein Testdatensatz von dem intelligenten System auf Fehlerstellen (23) untersucht wird, und wobei das Ergebnis dieser Untersuchung, insbesondere von dem intelligenten System, mit dem zugehörigen Ergebnisdatensatz verglichen wird.

5. Verfahren nach Anspruch 4, **dadurch gekennzeichnet, dass** das intelligente System seine Gewichtungen auf Basis des Vergleichs zwischen Ergebnis und Ergebnisdatensatz gegebenenfalls anpasst.

6. Verfahren nach einem der vorhergehenden Ansprüche, **dadurch gekennzeichnet, dass** zur Analyse der Ausrichtungsinformationen zur Erkennung der Fehlerstellen (23) des Körpers (11) eine Vielzahl, beispielsweise mehr als zehn, unterschiedliche Algorithmen verwendet werden, welche insbesondere in einem intelligenten System verwendet und unterschiedlich gewichtet werden können.

7. Verfahren nach einem der vorhergehenden Ansprüche, **dadurch gekennzeichnet, dass** für jede Beleuchtungssituation genaue eine Lichtquelle (16) angeschaltet ist und die gegebenenfalls anderen Lichtquellen (16) abgeschaltet sind.

8. Verfahren nach einem der vorhergehenden Ansprüche, **dadurch gekennzeichnet, dass** die Aufnahmeeinrichtung (15) und die Lichtquelle(n) (16) innerhalb einer haubenartigen, im wesentlichen lichtundurchlässigen Abdeckung (13) angeordnet sind, in oder unter welche der Körper (11) insbesondere eingefahren werden kann, beispielsweise durch einen klein gehaltenen Spalt (14) an der Unterseite der Abdeckung (13).

9. Verfahren nach einem der voranstehenden Ansprüche, **gekennzeichnet durch** den folgenden Verfahrensschritt:
- Darstellung der ermittelten Fehlerstellen auf einem Display und/oder dem Körper, beispielsweise durch eine Projektionseinheit.

10. Vorrichtung (10) zur automatischen Fehlerstellenerkennung bei biegeschlaffen Körpern (11), insbesondere bei Tierhäuten, umfassend eine, insbesondere mindestens eine Kamera (15a, b, c) aufweisende, Aufnahmeeinrichtung (15) sowie eine Steuerung (25), insbesondere eine Auflageeinheit (12) für den biegeschlaffen Körper (11) und eine oder mehrere Lichtquelle(n), wobei die Vorrichtung (10) derart eingerichtet ist, dass sie unter Verwendung von Aufnahmeeinrichtung (15), Steuerung (25) und einem neuronalen Netzwerk ein Verfahren nach einem der vorhergehenden Ansprüche ausführt.

## Claims

1. Method for detecting imperfections in flexible bodies (11), in particular animal skins, comprising the steps:
- providing one or more, in particular at least three, more particularly at least five, light source(s) (16), preferably spot light source(s), for illuminating at least one partial region of the body (11),
**characterised by** the following steps:
- producing a plurality of images of at least the partial region of the body (11) by means of an imaging device (15), in particular having at least one camera (15a, b, c),
- wherein each of the plurality of images is produced in a different illumination situation generated by the light source(s) (16), in particular in such a way that a different one of the plurality of light sources (16) is activated for each image,
- calculating alignment information, in particular the normal vector, of the smallest areas (30) associated with the body (11), for example pixels, on the basis of the plurality of images,
- analysing the alignment information for detecting imperfections in the body (11).

2. Method according to claim 1, **characterised in that** the position(s) of the light source(s) (16) and the imaging device (15) and/or brightness or colour values of the smallest areas (30) in the plurality of images are taken into account when calculating the alignment information, in particular in the manner of a photometric stereo method.

3. Method according to claim 1 or 2, **characterised in that** the analysis of the alignment information for detecting imperfections in the body (11) is carried out by an intelligent system, in particular in the manner of a neural network, more particularly in the manner of a convolutional neural network.

4. Method according to claim 3, **characterised in that** the intelligent system is trained by providing test data sets and associated result data sets, wherein a test data set is examined by the intelligent system for imperfections (23), and wherein the result of this examination is compared, in particular by the intelligent system, with the associated result data set.

5. Method according to claim 4, **characterised in that** the intelligent system adjusts its weightings, if necessary, on the basis of the comparison between the result and the result data set.

6. Method according to one of the preceding claims, **characterised in that** a plurality, for example more than ten, of different algorithms are used to analyse the alignment information to detect imperfections (23) in the body (11), which algorithms can be used in particular in an intelligent system and can be weighted differently.

7. Method according to one of the preceding claims, **characterised in that** for each lighting situation, exactly one light source (16) is switched on and the possible other light sources (16) are switched off.

8. Method according to one of the preceding claims, **characterised in that** the imaging device (15) and the light source(s) (16) are arranged within a hood-like, substantially opaque cover (13) into or under which the body (11) can in particular be retracted, for example through a small gap (14) on the underside of the cover (13).

9. Method according to one of the preceding claims, **characterised by** the following method step:
- depicting the determined imperfections on a display and/or the body, for example by means of a projection unit.

10. Device (10) for automatically detecting imperfections in flexible bodies (11), in particular animal skins, comprising an imaging device (15), in particular having at least one camera (15a, b, c), and a controller (25), in particular a support unit (12) for the flexible body (11) and one or more light source(s), wherein the device (10) is arranged in such a way that, using the imaging device (15), controller (25) and a neural network, it carries out a method according to one of the preceding claims.

## Revendications

1. Procédé pour la détection de défauts sur des corps flexibles (11), en particulier sur des peaux d'animaux, comprenant les étapes suivantes :
- fourniture d'une ou plusieurs, en particulier d'au moins trois, plus particulièrement d'au moins cinq sources lumineuses (16), de préférence des sources lumineuses ponctuelles pour éclairer au moins une zone partielle du corps (11),
**caractérisé par** les étapes suivantes :
- réalisation de plusieurs prises de vue au moins de la zone partielle du corps (11) au moyen d'un appareil de prise de vue (15) comportant au moins une caméra (15a, b, c),
- chacune des multiples prises de vue étant réalisée dans une autre situation d'éclairage par la/les source(s) lumineuse(s) (16), en particulier de telle sorte qu'une autre des multiples sources lumineuses (16) est activée pour chaque prise de vue,
- calcul d'informations d'orientation, en particulier du vecteur normal, de zones de base (30), par exemple de pixels, associées au corps (11), à l'aide des multiples prises de vue,
- analyse des informations d'orientation pour détecter des défauts du corps (11).

2. Procédé selon la revendication 1, **caractérisé en ce que**, lors du calcul des informations d'orientation, la/les position(s) de la/des source(s) lumineuse(s) (16) ainsi que de l'appareil de prise de vue (15) et/ou les valeurs de luminosité ou de couleur des zones de base (30) de plusieurs prises de vue sont prises en compte, en particulier à la manière d'un procédé stéréo-photométrique.

3. Procédé selon la revendication 1 ou 2, **caractérisé en ce que** l'analyse des informations d'orientation pour la détection des défauts du corps (11) est réalisée au moyen d'un système intelligent, en particulier à la manière d'un réseau neuronal, plus particulièrement à la manière d'un réseau neuronal convolutif.

4. Procédé selon la revendication 3, **caractérisé en ce que** le système intelligent est entraîné en générant des jeux de données de test et des jeux de données de résultat associés, un jeu de données de test étant analysé par le système intelligent sur des défauts (23) et le résultat de cette analyse étant comparé, en particulier par le système intelligent, avec le jeu de données de résultat associé.

5. Procédé selon la revendication 4, **caractérisé en ce que** le système intelligent adapte le cas échéant ses pondérations sur la base de la comparaison entre le résultat et le jeu de données de résultat.

6. Procédé selon une des revendications précédentes, **caractérisé en ce que** pour l'analyse des informations d'orientation pour détecter les défauts (23) du corps (11), une multitude d'algorithmes différents, par exemple plus de dix, sont utilisés, lesquels sont utilisés en particulier dans un système intelligent et peuvent être pondérés différemment.

7. Procédé selon une des revendications précédentes, **caractérisé en ce que** pour chaque situation d'éclairage, une seule source lumineuse (16) est allumée et les autres sources lumineuses (16) éventuelles sont éteintes.

8. Procédé selon une des revendications précédentes, **caractérisé en ce que** l'appareil de prise de vue (15) et la/les source(s) lumineuse(s) (16) sont disposés à l'intérieur d'un couvercle (13) en forme de capot sensiblement opaque, dans ou sous lequel le corps (11) peut en particulier être introduit, par exemple à travers une fente (14) de petite dimension dans la face inférieure du couvercle (13).

9. Procédé selon une des revendications précédentes, **caractérisé par** l'étape de procédé suivante :
- affichage des défauts constatés sur un écran et/ou sur le corps, par exemple au moyen d'une unité de projection.

10. Dispositif (10) pour la détection automatique de défaut sur des corps flexibles (11), en particulier sur des peaux d'animaux, comprenant un appareil de prise de vue (15) comportant en particulier au moins une caméra (15a, b, c), ainsi qu'une commande (25), en particulier une unité de support (12) pour le corps flexible (11) et une ou plusieurs sources lumineuses, lequel dispositif (10) est configuré de telle sorte qu'il met en œuvre un procédé selon une des revendications précédentes au moyen de l'appareil de prise de vue (15), de la commande (25) et d'un réseau neuronal.
